# EUROPEAN PATENT SPECIFICATION

(11) **EP 0 404 964 B1**
(45) Date of publication and mention of the grant of the patent: **10.08.1994**
(21) Application number: 90900978.9
(22) Date of filing: 15.12.1989
(51) Int. Cl.: C12P 19/44, C12P 19/16

(54) **PRODUCTION OF SUGAR COMPOUNDS**
VERFAHREN ZUR HERSTELLUNG VON ZUCKERVERBINDUNGEN
PROCEDE DE PRODUCTION DE COMPOSES DE SUCRE

(30) Priority: 16.12.1988 JP 316157/88
(43) Date of publication of application: 02.01.1991
(73) Proprietor: SHOWA DENKO KABUSHIKI KAISHA, Minato-ku, Tokyo (JP)
(72) Inventor: KITAKUNI, Eiichi, Showa Denko K.K., Ota-ku, Tokyo 146 (JP); HATTORI, Reiko, Showa Denko K.K., Ota-ku, Tokyo 146 (JP); SATO, Hajime, Showa Denko K.K., Ota-ku, Tokyo 146 (JP)
(74) Representative: Strehl Schübel-Hopf Groening & Partner
(86) International application number: JP8901259
(87) International publication number: WO9007002

(56) References cited:
- JP-A- 6 196 996
- US-A- 4 024 290
- US-A- 4 376 198
- WPIL, FILE SUPPLIER, Derwent Publications Ltd., London (GB); acc. no. 87337070 (48)#
- WPIL, FILE SUPPLIER, Derwent Publications Ltd., London (GB); acc. no. 88268275 (38)#
- CHEMICAL ABSTRACTS, vol. 69, no. 11, 09 September 1968, Columbus, OH (US); p. 4151, no. 44168q#

## Description

### TECHNICAL FIELD

The present invention relates to a process for the production of sugar compounds, characterized by bonding glucose and a sugar alcohol in the presence of α-glucosidase. More specifically, it relates to a process for easily and efficiently forming a sugar compound consisting of glucose and a sugar alcohol bonded to each other by an enzyme reaction, by coexisting glucose and a sugar alcohol such as sorbitol, mannitol, erythritol, xylitol, inositol, and allowing an action thereon by α-glucosidase.

### BACKGROUND ART

Sugar compounds consisting of glucose and a sugar alcohol bonded to each other are industrially produced by chemically reducing starch-derived sugars such as maltose or sugar syrup.

For example, α-D-glucopyranosyl-1,4-sorbitol is produced by chemically reducing maltose (maltose: 4-O-α-D-glucopyranosyl-D-glycopyranose) to convert glucose in a structural unit to sorbitol.

Moreover, α-D-glucopyranosyl-1,6-sorbitol can be produced by chemically reducing isomaltose (6-O-α-D-glucopyranosyl-D-glucopyranose); but since isomaltose per se. is very expensive, it is not always adequate as a starting material for the industrial production of α-D-glucopyranosyl-1,6-sorbitol.

On the other hand, α-D-glucopyranosyl-1,6-sorbitol and α-D-glucopyranosyl-1,6-mannitol can be also produced by chemically reducing a starting material isomalturose (α-D-glucopyranosyl-1,6-fructose) (Japanese Examined Patent Publication; KOKOKU, Nos. 57-9472 and 59-36694), but according to these processes, since α-D-glucopyranosyl-1,6-fructose as a starting material is produced from sucrose (α-D-glucopyranosyl-β-D-fructofranoside), an industrial process needs two steps starting from sucrose. Moreover, under a usual reducing condition, the product comprises a mixture of α-D-glucopyranosyl-1,6-sorbitol and α-D-glucopyranosyl-1,6-mannitol, and therefore, these processes are not always efficient as processes for the production of α-D-glucopyranosyl-1,6-sorbitol and α-D-glucopyranosyl-1,6-mannitol.

On the other hand, in a process for the production of a sugar compound by chemically bonding sugars to each other, it is generally difficult to selectively control a bonding site and molecular weight of a reaction product. Therefore, a process for a production of sugar compounds by a chemical bonding reaction is not always efficient from the viewpoint of industrial production.

Note, currently processes for the production of sugar compounds using enzymes are widely used in the starch industries. For example, hight fructose corn syrup is produced by an isomerization with glucose isomerase; fructooligosaccharide is produced by the transferase action of β-fructofranosidase; isomalturose is produced by the transferase action of by glucosyltransferase; and galactooligosaccharide is produced by the transferase action of β-galactosidase.

### DISCLOSURE OF THE INVENTION

As described above, although various processes for the production of sugar compounds, which are products formed by bonding glucose and a sugar alcohol, are known, they have drawbacks such as a high cost starting material, high cost of obtaining a highly purified product, because the reaction product is a mixture, and the like, and thus are not always industrially advantageous.

The present invention is intended to provide a completely novel process not having the above-mentioned drawbacks, which process is characterized by reacting glucose with a sugar alcohol in the presence of α-glucosidase.

According to the present invention, the starting materials are glucose and a sugar alcohol such as sorbitol, mannitol, erythritol, xylitol, inositol, which are relatively cheap and widely available. Moreover, according to the present invention characterized by bonding glucose with a sugar alcohol using α-glucosidase, since a sugar compound corresponding to a sugar alcohol as a starting material is predominantly obtained, a desired sugar compound is easily obtained in a pure form.

### BEST MODE OF CARRYING OUT THE INVENTION

In the present process, as the starting sugar alcohols, various reduced monosaccharides such as sorbitol, mannitol, erythritol, xylitol, inositol can be used, and corresponding to these sugar alcohols, target compounds such as α-D-glucopyranosyl-1,6-sorbitol, α-D-glucopyranosyl-1,6-mannitol, α-D-glucopyranosylerythritol, α-D-glucopyranosylxylitol, α-D-glucopyranosylinositol are obtained, respectively.

According to the present invention, the glucose and sugar alcohols used are not necessarily highly pure; for example, the glucose can contain impurities such as various oligosaccharides formed during the saccharification of starch.

Moreover, usually, sugar alcohols such as sorbitol and the like are produced by reducing sucrose, glucose, sugar syrup, xylose and the like, and thus the use of highly purified materials is not necessary in the present invention.

As the α-glucosidase used in the present process, any enzyme which cleaves an α-1,4-glycoside bond to form glucose can be used, and for example, enzymes produced by microorganisms belonging to the genus Streptomyces, Saccharomyces, or Bacillus, are known [J. Jpn. Soc. Starch Sci. Vol 31 (2), page 67, 1984; J. Jpn. Soc. Starch Sci. Vol 34 (4), page 292, 1987; Applied and Environmental Microbiology, page 1096, 1979; CAN. J. Microbiol. Vol 33, 1987; Biotechnology Letters Vol 5 (5), page 289; J. Jpn. Soc. Starch Sci. Vol 35 (1), page 69, 1988].

In the production of a sugar compound from glucose and a sugar alcohol according to the present process, first a reaction mixture containing glucose and a sugar alcohol in a relatively high concentration is prepared. The range of concentration of the starting materials in the reaction mixture is 5 to 100% by weight, preferably 20 to 100% by weight, to increase the amount of the sugar compound produced. The ratio of the sugar alcohol to glucose in the reaction mixture is approximately 100:1 to 1:100 by weight, more preferably 1:10 to 10:1, to increase the amount of the sugar compound produced. As the amount of an enzyme introduced to the reaction mixture is increased, the amount of the sugar compound produced in the Examples of the present invention is increased.

Reaction conditions such as the pH, temperature and the like according to the present invention may be such that the α-glucosidase maintains its enzymatic activity; for example, the pH value of the reaction mixture is adjusted to 4 to 10, more preferably 6 to 8. Regarding the reaction temperature, the reaction is preferably carried out at a relatively high temperature, as long as the stability of the enzyme is not lost; for example, preferably at 20°C to 70°C. But where a thermoresistant enzyme, or an alkaline enzyme or acid enzyme is used, the reaction conditions are not limited to the above-mentioned ranges.

In the present invention, the reaction using α-glucosidase is classified as follows. A batch system wherein the enzyme or microbial cells containing the enzyme are directly introduced into a reaction mixture, and a system wherein the enzyme or microbial cells containing the enzyme are immobilized, and a reaction mixture is applied thereon.

For example, where α-D-glucopyranosyl-1,6-sorbitol is formed from glucose and sorbitol, using the batch system according to the present invention, the concentration of glucose and sorbitol in a reaction mixture is made 10 to 100% by weight, more preferably 40 to 100% by weight. The ratio of sorbitol to glucose is 1:4 to 4:1 by weight, more preferably 2:3 to 3:2 by weight. The pH value of the reaction mixture is adjusted to 4 to 10, preferably 6 to 8, and for example, a commercially available α-glucosidase enzyme is directly added thereon to carry out a synthetic reaction. In this case, the reaction temperature is previously adjusted to 20 to 70°C, more preferably 40 to 60°C.

In the enzyme reaction described in Examples according to the present invention, an amount produced of a sugar compound, as represented by, for example, α-D-glucopyranosyl-1,6-sorbitol, increases with a longer reaction time, as long as the enzyme activity is maintained.

Further, surprisingly, the above-mentioned reaction selectively forms a sugar compound such as α-D-glucopyranosyl-1,6-sorbitol.

This means that sugar compounds such as α-D-glucopyranosyl-1,6-sorbitol can be produced very easily and selectively from glucose and a sugar alcohol, by using an enzyme reaction with α-glucosidase.

A sugar compound obtained according to the present process can be easily and efficiently recovered by eliminating the enzyme by, for example, using a membrane filter, etc., and separating α-D-glucosyl-l,6-sorbitol from starting materials such as glucose and sorbitol, etc., by using an active carbon or anion exchange resin.

### Examples

Next, the present invention is illustrated by Examples.

### Example 1.

A reaction mixture comprising 45% by weight glucose, 45% by weight sorbitol, 25 mM KH₂PO₄-K₂HPO₄ buffer (pH 7.4), and 5 mM Ca(NO₃)₂ was prepared in a test tube, which was then put into an incubator previously adjusted to 45°C, and then a commercially available α-glucosidase enzyme (Sigma) was added to this reaction mixture to bring a protein concentration to 0.1 - 500 mg/ml, thereby to start a reaction.

Examples were periodically obtained and an amount of α-D-glucopyranosyl-1,6-sorbitol was determined. As a result, where the protein concentration was 0.8 mg/ml, the amount of the product formed was 2.5% by weight 20 hours from the onset of the reaction, and 5.3% by weight 80 hours thereafter. On the other hand, where the protein concentration was 5.0 mg/ml, the amount of the product formed was 15.5% by weight 20 hours from the onset of the reaction, and 32.0% by weight 80 hours thereafter.

In the above-mentioned measurement, the sampled reaction mixture was heated to terminate the enzyme reaction, and filtered with a membrane filter by centrifugation to eliminate the enzyme. The protein-free reaction mixture was analyzed by high performance liquid chromatography (HPLC) and thin layer chromatography (TLC), and as a result, the product was confirmed to be same as a commercially available or chemically synthesized standard compound. Moreover, the structure of the product was analyzed, using NMR, and was confirmed to be same as a commercially available or chemically synthesized standard compound.

In the following Examples, products were analyzed in the same manner as described above.

### Example 2.

The same condition as in Example 1 was used except that, in place of glucose and sorbitol, 27% by weight of glucose and 27% by weight of mannitol were used, and the amount of α-glucopyranosyl-1,6-mannitol was measured. Where the protein concentration was 0.2 mg/ml, the amount was 0.5% after 20 hours, and 2.0% after 80 hours.

### Example 3.

The same condition as in Example 1 was used except that, in place of glucose and sorbitol, glucose (40%) and erythritol (40%), glucose (40%) and xylitol (40%), or glucose (25%) and inositol (25%) were used, and the amounts of sugar compound formed were measured. Where the protein concentration was 1.0 mg/ml, 80 hours after the onset of the reaction, glucopyranosylerythritol (7.0%), glucopyranosylxylitol (6.0%) and glucopyranosylinositol (4.5%) were formed, respectively. Note the numbers in parentheses show percent concentrations by weight.

### INDUSTRIAL APPLICABILITY

As sugar compounds formed by bonding glucose and a sugar alcohol according to the present invention, α-D-glucopyranosyl-1,6-sorbitol and α-D-glucopyranosyl-1,6-mannitol may be mentioned. These compound are characterized in that they have an anti-carious effect (low carious property) and a resistance to the digestion (low calorie), and therefore, are useful as sweetening materials.

Moreover, in addition to the above-mentioned characteristics, since they are less hygroscopic, they are suitable for use for chocolate, cookies, biscuits, and tabletted cake. Moreover, in contrast to sugar syrup, maltose and the like, since the sugar compounds are thermostable, and the time for the addition and the heating temperature are not limited, they are advantageous in that the workability can be improved.

As seen from the above, the sugar compounds consisting of glucose and a sugar alcohol bonded to each other have various useful properties, from the point of view of the industrial applicability thereof, in comparison with starch-derived sugars such as glucose, sugar syrup, maltose, powdered malt dextrin, high fructose corn syrup and the like. Moreover, since these sugar compounds are less assimilable with fungi (Penicillium, yellow Aspersillus, black Aspergillus, Mucor) and yeast as well as enzymes, when used in lactic acid-containing drinks, fermentation foods, as well as wet foods such as pickles, cakes and the like, they inhibit fermentation, prolong the product life, and improve the storage stability of the product.

## Claims

1. A process for the production of a sugar compound characterized by bonding glucose and a sugar alcohol in the presence of α-glucosidase.

2. A process according to claim 1, wherein the sugar compound is an α-D-glucopyranosyl sugar alcohol.

3. A process according to claim 1, wherein sorbitol, mannitol, erythritol, xylitol or inositol is used as the sugar alcohol, and the corresponding α-D-glucopyranosyl-1,6-sorbitol, α-D-glucopyranosyl-1,6-mannitol, α-D-glucopyranosylerythritol, α-D-glucopyranosylxylitol or α-D-glucopyranosylinositol is produced, respectively.

4. A process according to claim 1, wherein the α-glucosidase is that produced by a microorganism belonging to the genus Streptomyces, Saccharomyces or Bacillus.

## Patentansprüche

1. Verfahren zur Herstellung einer Zuckerverbindung, welches durch das Verbinden von Glucose und einem Zuckeralkohol in Gegenwart von α-Glucosidase gekennzeichnet ist.

2. Verfahren gemäß Anspruch 1, worin die Zuckerverbindung ein α-D-Glucopyranosyl-Zuckeralkohol ist.

3. Verfahren gemäß Anspruch 1, worin Sorbit, Mannit, Erythrit, Xylit oder Inosit als Zuckeralkohol verwendet wird, und jeweils das entsprechende α-D-Glucopyranosyl-1,6-sorbit, α-D-Glucopyranosyl-1,6-mannit, α-D-Glucopyranosylerithrit, α-D-Glucopyranosylxylit oder α-D-Glucopyranosylinosit hergestellt wird.

4. Verfahren gemäß Anspruch 1, worin die α-Glucosidase eine durch einen zum Genus Streptomyces, Saccharomyces oder Bacillus gehörenden Microorganismus hergestellte ist.

## Revendications

1. Un procédé pour la production de composés de sucre, caractérisé par la liaison de glucose et d'un alcool de sucre en présence d'une α-glucosidase.

2. Un procédé suivant la revendication 1, dans lequel le composé de sucre est un α-O-glucopyranosyl-alcool de sucre.

3. Un procédé suivant la revendication 1, dans lequel le sorbitol, le mannitol, l'érythrol, le xylitol ou l'inositol est utilisé comme alcool de sucre et il est produit respectivement α-O-glucopyranosyl-1,6-sorbitol, α-O-glucopyranosyl-1,6-mannitol, α-O-glucopyranosylérythritol, α-O-glucopyranosylxylitol ou α-O-glucopyranosylinositol.

4. Un procédé suivant la revendication 1, dans lequel l'α-glucosidase est celle produite par un micro-organisme appartenant aux espèces des Streptomyces, Saccharomyces, ou Bacillus.
